# FASCICULE DE BREVET EUROPEEN

(11) **EP 3 223 916 B1**
(45) Date de publication et mention de la délivrance du brevet: **09.01.2019**
(21) Numéro de dépôt: 15820054.3
(22) Date de dépôt: 26.11.2015
(51) Int. Cl.: A62B 17/00, A62B 17/04, A41D 13/11, A61F 9/02

(54) **CAGOULE DE PROTECTION DE TÊTE**
KOPFSCHUTZHAUBE
HEAD PROTECTIVE HOOD

(30) Priorité: 28.11.2014 EP 14195539
(43) Date de publication de la demande: 04.10.2017
(73) Titulaire: Scaldis St-Martin, 7600 Peruwelz (BE)
(72) Inventeur: VANNESTE, Vincent, 7533 Thimougies (BE)
(74) Mandataire: Calysta NV
(86) Numéro de dépôt international: PCT/EP2015/077753
(87) Numéro de publication internationale: WO 2016/083496

(56) Documents cités:
- FR-A1- 2 928 260
- US-A1- 2002 124 299
- US-A1- 2010 229 858

## Description

La présente invention se rapporte à une cagoule de protection de tête faisant partie intégrante d'un vêtement de protection pour salle blanche, ladite cagoule présentant :
- une face extérieure prévue pour être en contact avec un milieu environnant,
- une face intérieure prévue pour être en contact avec la tête d'un utilisateur,
- une section de passage de tête,
- une ouverture faciale prévue au niveau des yeux de l'utilisateur, et
- un premier et un deuxième moyens de liaison de lunette de protection, lesdits moyens de liaison étant reliés à ladite cagoule respectivement de part et d'autre de ladite ouverture faciale.

Une telle cagoule est connue du document FR2928260 où les sangles permettant initialement d'ajuster la cagoule au niveau de la tête d'un utilisateur (par nouage de ces sangles à l'arrière de la tête) sont également utilisées pour être passées dans des boucles présentes au niveau d'une lunette de protection lors de l'habillage. Ces sangles, selon ce document antérieur, constituent donc des moyens de liaison utilisés en cours d'habillage pour relier une lunette de protection (formant en soi un équipement individuel) au niveau de la cagoule d'une combinaison pour salle blanche.

Toutefois, le procédé de mise en place d'une lunette de protection au niveau d'une cagoule d'une combinaison selon ce document antérieur, même s'il permet de minimiser le nombre de fois où l'utilisateur touche la lunette, ne réduit pas les risques de chute de la lunette lorsqu'il convient de procéder au passage des sangles dans les boucles (anneaux) de la lunette. Par ailleurs, lors du placement de la lunette de protection, les sangles doivent ensuite être nouées à l'arrière de la tête, ce qui n'est pas aisé. Qui plus est, si par inadvertance ou suite à une erreur de manipulation, l'utilisateur lâche les sangles ou si ces dernières lui glissent entre les doigts, il y a de fortes chances pour que la lunette de protection tombe sur le sol. Il en résulterait une contamination de cette dernière, ce qui est intolérable dans le domaine des salles blanches et la procédure d'habillage devrait être au moins recommencée en partie, au moins en ce qui concerne le placement de la lunette de protection.

Ce type de cagoule est particulièrement utilisé dans des environnements aseptiques et stériles où l'utilisateur doit être isolé du milieu environnant. Ceci est particulièrement indiqué dans des environnements tels que les salles blanches et les laboratoires où se préparent vaccins et médicaments. D'une part, dans ce type d'environnement, il convient d'isoler au mieux l'utilisateur afin qu'il ne contamine pas l'environnement dans lequel il travaille et, d'autre part, il s'agit souvent d'isoler l'utilisateur lui-même de substances dangereuses et/ou nocives.

Lorsqu'un opérateur est amené à travailler dans un environnement stérile, il doit particulièrement veiller à ne pas contaminer l'équipement qu'il revêt. Ainsi, l'opérateur doit respecter des procédures d'habillage strictes, lesquelles n'ont de sens que si l'équipement de protection qu'il utilise est lui-même tout à fait stérile. A cette fin, les combinaisons et les cagoules sont généralement fournies après stérilisation, dans des emballages étanches dont une ouverture aisée permet une saisie des équipements stériles.

Afin de protéger les yeux de l'opérateur, des cagoules actuellement utilisées sont munies d'une visière fixe ou amovible pour assurer l'isolation et la protection de l'utilisateur mais aussi pour minimiser les risques de contamination du milieu environnant (vaccins, médicaments, ...) (voir par exemple la cagoule de protection E5111101 commercialisée par Protextyl®). Toutefois, plusieurs inconvénients sont inhérents aux cagoules munies d'une visière. En effet, la présence d'une visière donne lieu à un champ de vision restreint (présence d'un angle mort) et à la formation de buée sur la visière. C'est pourquoi, le recours à des lunettes de protection reste préféré à ce jour.

Cette approche de recours à des lunettes de protection consiste classiquement à utiliser des cagoules pouvant faire partie intégrante d'une combinaison pour salle blanche, par-dessus lesquelles sont positionnées des lunettes de protection de type « masque de ski » (googles) (comme par exemple celles décrites dans le document EP0195517). Ces lunettes de protection constitue donc un équipement individuel et additionnel que l'opérateur doit absolument manipuler précautionneusement pour le pas les laisser tomber, auquel cas il conviendrait de changer de lunettes de protection puisqu'elles auraient été contaminées. Par ailleurs, un positionnement correct de ces lunettes de protection n'est pas aisé puisque qu'il repose généralement sur la mise en place d'une bande élastique qui entoure la tête de l'opérateur. L'opérateur doit donc tendre cette bande élastique, passer les lunettes par-dessus sa tête puis seulement procéder à l'ajustement des lunettes de protection au niveau de ses yeux.

Toutes ces étapes de mise en place des lunettes de protection augmentent chacune les risques d'erreur de manipulation (chute des lunettes de protection, apport de contaminants sur l'écran de la lunette, ...) et par conséquent les risques de contamination. Notons que, comme indiqué plus haut concernant le document FR2928260, cette même problématique est observée pour des lunettes de protection dont la mise en place repose sur le nouage d'une sangle à l'arrière de la tête, cette opération étant encore plus contraignante que dans le cas d'une bande élastique, les risques de chute et de contamination étant donc d'autant plus importants.

Il est donc clair, qu'à ce jour, le recours à des lunettes comme moyen de protection des yeux, même s'il reste le moyen préféré, est contraignant, repose sur de nombreuses manipulations des lunettes et de leur système de fixation (bande élastique, bande à nouer, ...), ralentit les procédures d'habillage et augmente considérablement les risques de contamination. En effet, la probabilité de chute des lunettes de protection est présente et les nombreuses manipulations requises pour leur mise en place augmentent de façon non négligeable les risques de transfert de contaminants depuis l'opérateur sur les lunettes de protection et ensuite vers le milieu environnant. En outre, cette approche de l'état de la technique implique généralement un conditionnement stérile séparé de la cagoule et des lunettes de protection, ces deux éléments ne pouvant généralement pas être soumis aux mêmes conditions de stérilisation. L'opérateur doit donc ouvrir deux contenants différents, ce qui, à nouveau, augmente de façon non négligeable les risques de transfert de contaminants.

Il existe donc un réel besoin de simplifier l'utilisation des cagoules actuelles pouvant faire partie intégrante d'une combinaison pour salle blanche et utilisées en association avec des lunettes de protection (de type masque de ski) afin que les étapes de revêtement d'équipements (de vêtements) de protection et de lunettes de protection soient moins contraignantes, plus rapides et plus sûres afin de minimiser tout risque de contamination.

L'invention a pour but de pallier les inconvénients de l'état de la technique en procurant une cagoule faisant partie intégrante d'un vêtement de protection pour salle blanche, ladite cagoule permettant de simplifier le placement et l'utilisation de lunettes de protection, de telle façon à rendre le placement de ces dernières simple et rapide en minimisant leur manipulation par l'opérateur qui revêt la cagoule de protection, ceci tout en assurant une minimisation des risques de contamination tant de l'opérateur que du milieu environnant.

Pour résoudre ces problèmes, il est prévu suivant l'invention, une cagoule telle qu'indiquée au début, caractérisée en ce que, à l'état emballé et lors de l'habillage, une lunette de protection (L) est retenue à la cagoule par au moins un desdits premier et deuxième moyens de liaison.

Selon l'invention, au moins un moyen de liaison est relié à la cagoule de chaque côté de l'ouverture faciale, ledit au moins un moyen de liaison retenant une lunette de protection sur la cagoule lorsque cette dernière, faisant partie intégrante d'un vêtement de protection pour salle blanche, est emballée (c'est-à-dire avant l'habillage et dans son conditionnement stérile) mais aussi lors de l'habillage (c'est-à-dire lors de l'enfilage de la combinaison). Par exemple, ledit au moins un moyen de liaison retient la lunette sur la cagoule en assurant un lien entre la cagoule et la monture ou le cadre de la lunette de protection.

De par la présence de moyens de liaison de part et d'autre de l'ouverture faciale et dès lors que la lunette est retenue par ces moyens de liaison dès le départ (c'est-à-dire déjà lorsque la combinaison et donc la cagoule en faisant partie intégrante sont dans un emballage stérile avant utilisation), l'opérateur qui revêt le vêtement de protection (la combinaison) et donc la cagoule suivant l'invention ne doit pas se soucier du maintien de la lunette. L'opérateur a les mains libres et peut positionner correctement la cagoule sur sa tête sans se préoccuper de la lunette de protection qui est retenue et qui ne risque donc pas de tomber.

En outre, lorsque la lunette de protection est retenue par les moyens de liaison dès le conditionnement de la combinaison et donc de la cagoule dans un emballage stérile, l'opérateur n'a pas à saisir la lunette de protection pour l'amener au niveau de son visage et plus particulièrement devant l'ouverture faciale de la cagoule, ce qui élimine une étape d'habillage sensible durant laquelle peuvent avoir lieu facilement des contaminations et ce qui restreint donc le risque de contamination. En effet, si la lunette est retenue sur la cagoule dès le départ, c'est-à-dire avant même que l'opérateur ne saisisse la cagoule, il n'a pas à ouvrir un autre contenant stérile qui contiendrait uniquement la lunette et il n'a pas à appliquer une procédure d'habillage complexe consistant à passer une bande élastique autour de sa tête ou à nouer une bande à l'arrière de sa tête pour assurer la mise en place et le maintien des lunettes de protection, ces opérations étant propices à l'établissement de contamination.

Selon l'invention, la cagoule munie de moyens de liaison de part et d'autre de l'ouverture faciale permet donc de réduire le nombre d'étapes d'habillage et permet aussi de conditionner la cagoule (faisant partie intégrante d'un vêtement de protection pour salle blanche) et la lunette de protection dans un même emballage stérile, la lunette étant déjà positionnée correctement au niveau de la cagoule, tout ceci contribuant à minimiser significativement les risques de contamination.

Avantageusement, selon l'invention, ledit un premier moyen de liaison et/ou un deuxième moyen de liaison reliés à ladite cagoule sont situés dans un axe qui traverse sensiblement horizontalement ladite ouverture faciale. Un tel positionnement de chaque moyen de liaison relié à la cagoule de part et d'autre de l'ouverture faciale permet que la lunette soit retenue et positionnée dans une position qui minimise la manipulation et le déplacement de la lunette lors de son placement correct de telle façon à couvrir l'ouverture faciale et à protéger les yeux de l'opérateur. Ce positionnement dans un axe qui traverse sensiblement horizontalement l'ouverture faciale contribue donc également à minimiser et à simplifier la procédure d'habillage en réduisant par conséquent les risques de contamination.

De préférence, selon l'invention, ledit un premier moyen de liaison et/ou un deuxième moyen de liaison sont reliés à ladite face extérieure ou à ladite face intérieure de la cagoule. Lorsque le moyen de liaison est relié à la face extérieure de la cagoule, il peut par exemple être cousu mais peut aussi être relié à la cagoule par tout autre moyen de fixation adéquat. Une fixation à la face interne de la cagoule est également possible selon l'invention mais, dans ce cas, une fente devrait être prévue à proximité des rebords latéraux de l'ouverture faciale pour que le moyen de liaison puisse y passer et se retrouver au moins partiellement à l'extérieur de la cagoule.

Avantageusement, selon l'invention, ledit un premier moyen de liaison et/ou un deuxième moyen de liaison sont reliés à une partie arrière de ladite cagoule ou à une partie latérale de ladite cagoule.

De préférence, selon l'invention, ledit un premier moyen de liaison et/ou un deuxième moyen de liaison présentent une première extrémité reliée à ladite cagoule et une deuxième extrémité libre.

Il est en effet tout à fait possible de relier le moyen de liaison soit à l'arrière de la cagoule, soit sur une partie latérale de la cagoule. Dans les deux cas, une première extrémité du moyen de liaison est reliée à la cagoule tandis qu'une deuxième extrémité libre est agencée pour être située latéralement par rapport à l'ouverture faciale.

Préférentiellement, selon l'invention, ledit un premier moyen de liaison et/ou un deuxième moyen de liaison comprennent un organe d'engagement agencé pour être engagé dans un organe d'engagement correspondant relié à une lunette de protection.

Avantageusement, selon l'invention, ledit organe d'engagement comprend une extrémité mâle ou femelle agencée pour être engagée dans un organe d'engagement correspondant comprenant une extrémité mâle ou femelle correspondante reliée à une lunette de protection.

Tout moyen de liaison relié à la cagoule et permettant de retenir une lunette, par exemple au niveau de la monture de cette dernière, peut être envisagé suivant la présente invention. Il pourrait par exemple s'agir d'un système de boucles ou plus simplement d'un élastique.

De préférence, selon l'invention, ledit au moins un moyen de liaison est ajustable en longueur. Un réglage en longueur du moyen de liaison permet d'assurer un positionnement adéquat de la lunette de protection au niveau de l'ouverture faciale de telle sorte que la lunette épouse le visage de l'opérateur et/ou les rebords de l'ouverture faciale, ce qui isole l'opérateur du milieu environnant de façon optimale. Par exemple, si le moyen de liaison est un système de boucle, une traction sur la partie libre de moyen de liaison passant dans ce système de boucle permet d'ajuster le positionnement de la lunette.

Avantageusement, la cagoule suivant l'invention comprend un système d'auto-ajustement de la taille de la cagoule placé à l'arrière de la cagoule, par exemple des boutons pressions permettant de régler la largeur de la cagoule et/ou la hauteur de la cagoule. En choisissant de fixer un bouton pression sur un point d'attache plutôt qu'un autre, il est possible de définir une taille de la cagoule adaptée à la morphologie de l'utilisateur de telle sorte que la cagoule épouse correctement la tête de l'opérateur et soit positionnée de manière à ne pas gêner l'opérateur.

D'autres systèmes d'auto-ajustement comme par exemple des liens ou des sangles peuvent également permettre d'ajuster la cagoule à la morphologie de l'opérateur. Par conséquent, une cagoule suivant l'invention peut convenir à plusieurs utilisateurs qui peuvent en adapter la taille. Il est bien entendu que tout autre moyen d'auto-ajustement adéquat peut être utilisé dans le cadre de la présente invention.

De préférence, la cagoule suivant l'invention fait partie intégrante d'un vêtement de protection qui est une combinaison telle que divulguée dans le document EP2303044, cette combinaison comprenant une face extérieure prévue pour être en contact avec un milieu environnant et une face intérieure agencée pour être en contact avec un manipulateur, une première et une deuxième section de jambe reliées l'une à l'autre par une ouverture dans une zone d'entrejambe (cette ouverture étant prévue pour être refermée de telle sorte que la zone d'entrejambe présente deux sections de jambe fermées, une première et une deuxième manche et une partie de corps à laquelle sont reliées les premières et deuxièmes manches par une extrémité liée et les première et deuxième section de jambe également par une extrémité liée, ladite partie de corps comprenant une section de passage de tête, ladite combinaison comprenant en outre au moins une zone de préhension en contact avec la face intérieure dans la partie de corps.

Ladite au moins une zone de préhension d'une telle combinaison peut par exemple être une lichette, un élastique, une patte, un cordon ou peut encore être formée par une zone du tissu (qui ne doit pas être formellement délimitée) de la combinaison, cette zone étant agencée pour être agrippée et saisie.

Avantageusement, la cagoule de protection de tête selon l'invention fait partie intégrante d'un vêtement de protection étant une combinaison dont l'ouverture est située dans la partie de corps, par exemple au centre du thorax ou depuis l'épaule jusqu'à la hanche d'un opérateur.

D'autres formes de réalisation de la cagoule suivant l'invention sont indiquées dans les revendications annexées.

La présente invention porte également sur un ensemble comprenant :
- une cagoule de protection de tête selon l'invention faisant partie intégrante d'un vêtement de protection pour salle blanche, et
- une lunette de protection retenue par au moins un desdits premier et deuxième moyen de liaison reliés à ladite cagoule, à l'état emballé et lors de habillage.

D'autres formes de réalisation de l'ensemble suivant l'invention sont indiquées dans les revendications annexées.

La présente invention porte également sur un procédé d'habillage d'un vêtement de protection pour salle blanche muni d'une cagoule de protection de tête selon l'invention, ledit procédé comprenant les étapes de :
- un enfilage, par un utilisateur, du vêtement de protection pour salle blanche par une ouverture de ce dernier, avec positionnement de la tête de l'utilisateur dans ladite cagoule, une lunette de protection étant retenue à la cagoule par au moins un desdits premier et deuxième moyen de liaison, et
- un ajustement, par l'utilisateur, du positionnement de la lunette de protection devant ladite ouverture faciale prévue au niveau de ses yeux.

Avantageusement, le procédé suivant l'invention comprend en outre une étape de liaison de la lunette de protection à la cagoule par un deuxième moyen de liaison lorsque la lunette de protection n'est préalablement retenue à la cagoule que par un premier moyen de liaison.

De préférence, le procédé suivant l'invention comprend une étape additionnelle de fermeture de ladite ouverture du vêtement de protection pour salle blanche, par exemple par l'intermédiaire d'une tirette ou de tout autre moyen de fermeture adéquat.

D'autres formes de réalisation du procédé suivant l'invention sont indiquées dans les revendications annexées.

D'autres caractéristiques, détails et avantages de l'invention ressortiront de la description donnée ci-après, à titre non limitatif et en faisant référence aux dessins annexés.

La figure 1 est une vue de face d'une cagoule de protection de tête faisant partie intégrante d'un vêtement de protection selon l'invention.

La figure 2 illustre une vue de côté d'une cagoule de protection de tête faisant partie intégrante d'un vêtement de protection selon l'invention.

Les figures 3a, 3b et 3c illustrent une cagoule suivant l'invention qui est reliée et fait partie intégrante d'un vêtement de protection.

Sur les figures, les éléments identiques ou analogues portent les mêmes références.

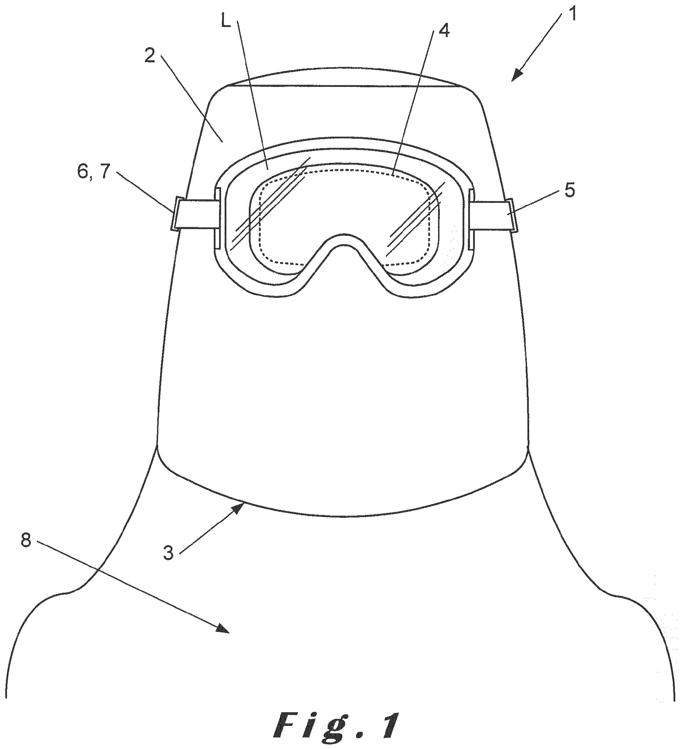

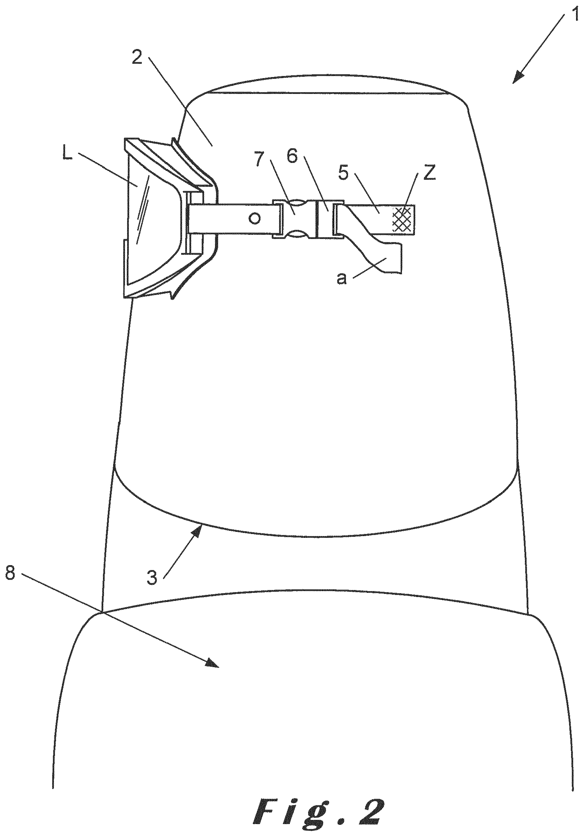

La figure 1 illustre une cagoule 1 suivant l'invention faisant partie intégrante d'un vêtement de protection 8 pour salle blanche et qui présente une face extérieure 2 prévue pour être en contact avec le milieu environnant, une face intérieure prévue pour être en contact avec la tête d'un utilisateur, une section de passage de tête 3 et une ouverture faciale 4 au niveau des yeux de l'utilisateur. En outre, comme illustré à la figure 2, la cagoule 1 comprend, de part et d'autre de l'ouverture faciale 4, au moins un moyen de liaison 5 agencé pour retenir une lunette de protection L. Ce moyen de liaison 5 présente une extrémité z reliée à la cagoule 1 et une extrémité libre a qui comprend un organe d'engagement 6 (ici un organe d'engagement de type mâle). Cet organe d'engagement 6 est agencé pour permettre un emboitement d'un organe d'engagement 7 correspondant (ici un organe d'engagement de type femelle) présent au niveau d'une lunette de protection L.

Sur la figure 1, l'organe d'engagement 7 présent au niveau de la lunette de protection L est relié à la monture de la lunette par l'intermédiaire d'un moyen de liaison. Il est bien entendu que ce moyen de liaison n'est pas indispensable et que l'organe d'engagement 7 pourrait être directement présent sur la monture sans qu'il y ait besoin d'un moyen de liaison.

En outre, le moyen de liaison 5 est réglable en longueur, ce qui permet d'amener la lunette de protection en contact avec les rebords de l'ouverture faciale 4 et/ou en contact avec une partie du visage de l'opérateur, de telle sorte que ce dernier est protégé adéquatement au niveau des yeux. Ici, le réglage en longueur du moyen de liaison s'effectue en tirant sur l'extrémité de l'extrémité libre du moyen de liaison passant au travers de l'organe d'engagement 6. Ceci est typiquement le cas lorsque l'organe d'engagement 6 se présente sous la forme d'un système de boucle. Il est bien entendu que tout autre type de moyen d'engagement adéquat fait partie du cadre de la présente invention.

Eventuellement, la cagoule 1, faisant partie intégrante d'un vêtement de protection 8 pour salle blanche, peut présenter une ou plusieurs zones formées dans un matériau différent que celui de la face extérieure de la cagoule 1, par exemple en un matériau perméable. De telles zones peuvent être situées en-dessous et/ou au-dessus de l'ouverture faciale 4 afin de permettre à l'opérateur de respirer aisément mais aussi afin d'aérer l'intérieur de la cagoule 1.

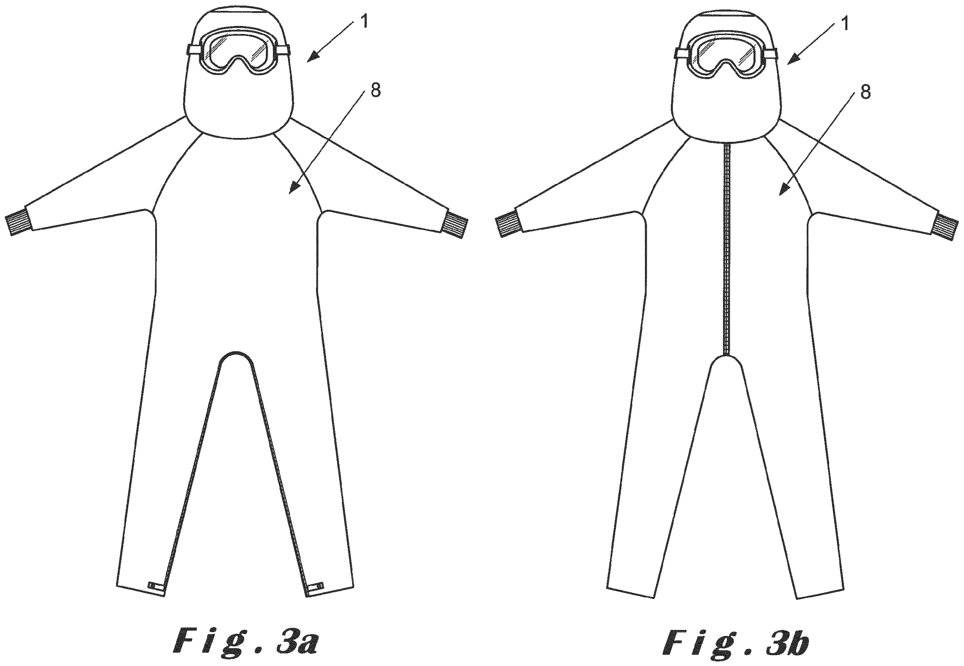

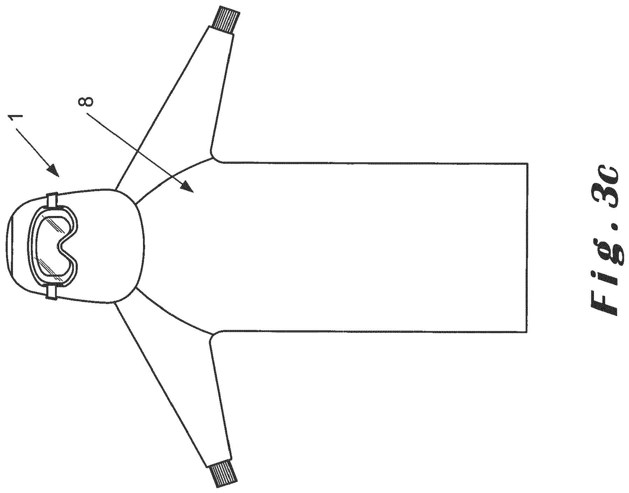

Les figures 3a, 3b et 3c illustrent une cagoule 1 qui est reliée et fait partie intégrante d'un vêtement de protection 8, en l'occurrence, pour l'exemple, d'une combinaison pour salles blanches (figures 3a et 3b) et analogues (blouse ou casaque en figure 3c). A la figure 3b, la tirette est représentée au niveau du thorax de l'opérateur mais elle pourrait tout à fait être située au niveau du dos de ce dernier.

Il est bien entendu que la cagoule 1 peut être reliée par tout moyen de fixation adéquat au vêtement de protection 8 de telle façon a en faire partie intégrante, par exemple en y étant cousue ou en y étant fixée à l'aide d'une tirette, de boutons à pression ou encore à l'aide de Velcro®.

Il est bien entendu que la présente invention n'est en aucune façon limitée aux formes de réalisations décrites ci-dessus et que bien des modifications peuvent y être apportées sans sortir du cadre des revendications annexées.

## Revendications

1. Cagoule (1) de protection de tête faisant partie intégrante d'un vêtement de protection (8) pour salle blanche, ladite cagoule (1) présentant :
- une face extérieure (2) prévue pour être en contact avec un milieu environnant,
- une face intérieure prévue pour être en contact avec la tête d'un utilisateur,
- une section de passage de tête (3),
- une ouverture faciale (4) prévue au niveau des yeux de l'utilisateur, et
- un premier et un deuxième moyens de liaison (5) de lunette de protection (L), lesdits moyens de liaison (5) étant reliés à ladite cagoule (1) respectivement de part et d'autre de ladite ouverture faciale (4),
ladite cagoule étant **caractérisée en ce que**, à l'état emballé et lors de l'habillage, une lunette de protection (L) est retenue à la cagoule (1) par au moins un desdits premier et deuxième moyens de liaison.

2. Cagoule (1) de protection de tête selon la revendication 1, **caractérisée en ce que** ledit un premier moyen de liaison et/ou un deuxième moyen de liaison (5) reliés à ladite cagoule (1) sont situés dans un axe qui traverse sensiblement horizontalement ladite ouverture faciale (4).

3. Cagoule (1) de protection de tête selon la revendication 1 ou 2, **caractérisée en ce que** ledit un premier moyen de liaison et/ou un deuxième moyen de liaison sont reliés à ladite face extérieure (2) ou à ladite face intérieure de la cagoule (1).

4. Cagoule (1) de protection de tête selon l'une quelconque des revendications précédentes, **caractérisée en ce que** ledit un premier moyen de liaison et/ou un deuxième moyen de liaison (5) sont reliés à une partie arrière de ladite cagoule (1) ou à une partie latérale de ladite cagoule (1).

5. Cagoule (1) de protection de tête selon l'une quelconque des revendications précédentes, **caractérisée en ce que** ledit un premier moyen de liaison et/ou un deuxième moyen de liaison (5) présentent une première extrémité (z) reliée à ladite cagoule (1) et une deuxième extrémité libre (a).

6. Cagoule (1) de protection de tête selon l'une quelconque des revendications précédentes, **caractérisée en ce que** ledit un premier moyen de liaison et/ou un deuxième moyen de liaison (5) comprennent un organe d'engagement (6) agencé pour être engagé dans un organe d'engagement (7) correspondant relié à une lunette de protection (L).

7. Cagoule (1) de protection de tête selon la revendication 6, **caractérisée en ce que** ledit organe d'engagement (6) comprend une extrémité mâle ou femelle agencée pour être engagée dans un organe d'engagement correspondant (7) comprenant une extrémité mâle ou femelle correspondante reliée à une lunette de protection (L).

8. Cagoule (1) de protection de tête selon l'une quelconque des revendications précédentes, **caractérisée** en ce ledit au moins un moyen de liaison (5) est ajustable en longueur.

9. Cagoule (1) de protection de tête selon l'une quelconque des revendications précédentes, **caractérisée en ce qu'**elle comprend un système d'auto-ajustement de la taille de la cagoule placé à l'arrière de la cagoule.

10. Ensemble comprenant une :
- une cagoule (1) de protection de tête faisant partie intégrante d'un vêtement de protection (8) pour salle blanche selon l'une quelconque des revendications 1 à 9, et
- une lunette de protection (L) retenue par au moins un desdits premier et deuxième moyen de liaison (5) reliés à ladite cagoule (1), à l'état emballé et lors de habillage.

11. Procédé d'habillage d'un vêtement de protection (8) pour salle blanche muni d'une cagoule de protection de tête selon l'une quelconque des revendications 1 à 9, ledit procédé comprenant les étapes de :
- un enfilage, par un utilisateur, du vêtement de protection (8) pour salle blanche par une ouverture de ce dernier, avec positionnement de la tête de l'utilisateur dans ladite cagoule (1), une lunette de protection (L) étant retenue à la cagoule (1) par au moins un desdits premier et deuxième moyen de liaison (5), et
- un ajustement, par l'utilisateur, du positionnement de la lunette de protection (L) devant ladite ouverture faciale (4) prévue au niveau de ses yeux.

12. Procédé d'habillage selon la revendication 11, comprenant en outre une étape de liaison de la lunette de protection (L) à la cagoule (1) par un deuxième moyen de liaison (5) lorsque la lunette de protection (L) n'est préalablement retenue à la cagoule (1) que par un premier moyen de liaison (5).

13. Procédé selon la revendication 11 ou 12 comprenant en outre une étape additionnelle de fermeture de ladite ouverture du vêtement de protection (8) pour salle blanche, par exemple par l'intermédiaire d'une tirette ou de tout autre moyen de fermeture adéquat.

## Patentansprüche

1. Kopfschutzhaube (1), die integraler Bestandteil einer Schutzkleidung (8) für Reinräume ist, wobei die Haube (1) aufweist:
- eine Außenseite (2), die dafür vorgesehen ist, sich mit einem Umgebungsmedium in Kontakt zu befinden,
- eine Innenseite, die dafür vorgesehen ist, sich mit dem Kopf eines Benutzers in Kontakt zu befinden,
- einen Kopf-Durchführabschnitt (3),
- eine Gesichtsöffnung (4), die im Bereich der Augen des Benutzers vorgesehen ist, und
- ein erstes und ein zweites Verbindungsmittel (5) für eine Schutzbrille (L), wobei die Verbindungsmittel (5) jeweils auf beiden Seiten der Gesichtsöffnung (4) mit der Haube (1) verbunden sind,
wobei die Haube **dadurch gekennzeichnet ist, dass** im verpackten Zustand und beim Anziehen eine Schutzbrille (L) von mindestens einem der ersten und zweiten Verbindungsmittel an der Haube (1) gehalten wird.

2. Kopfschutzhaube (1) nach Anspruch 1, **dadurch gekennzeichnet, dass** das eine erste Verbindungsmittel und/oder eine zweite Verbindungsmittel (5), die mit der Haube (1) verbunden sind, in einer Achse liegen, die im Wesentlichen horizontal durch die Gesichtsöffnung (4) verläuft.

3. Kopfschutzhaube (1) nach Anspruch 1 oder 2, **dadurch gekennzeichnet, dass** das eine erste Verbindungsmittel und/oder eine zweite Verbindungsmittel mit der Außenseite (2) oder mit der Innenseite der Haube (1) verbunden sind.

4. Kopfschutzhaube (1) nach einem der vorstehenden Ansprüche, **dadurch gekennzeichnet, dass** das eine erste Verbindungsmittel und/oder eine zweite Verbindungsmittel (5) mit einem hinteren Teil der Haube (1) oder mit einem seitlichen Teil der Haube (1) verbunden sind.

5. Kopfschutzhaube (1) nach einem der vorstehenden Ansprüche, **dadurch gekennzeichnet, dass** das eine erste Verbindungsmittel und/oder eine zweite Verbindungsmittel (5) ein erstes Ende (z), das mit der Haube (1) verbunden ist, und ein zweites, freies Ende (a) aufweisen.

6. Kopfschutzhaube (1) nach einem der vorstehenden Ansprüche, **dadurch gekennzeichnet, dass** das eine erste Verbindungsmittel und/oder eine zweite Verbindungsmittel (5) ein Eingriffsglied (6) umfassen, das dafür eingerichtet ist, in einem entsprechenden Eingriffsglied (7) in Eingriff gebracht zu werden, das mit einer Schutzbrille (L) verbunden ist.

7. Kopfschutzhaube (1) nach Anspruch 6, **dadurch gekennzeichnet, dass** das Eingriffsglied (6) ein männliches oder weibliches Ende umfasst, das dafür eingerichtet ist, in einem entsprechenden Eingriffsglied (7) in Eingriff gebracht zu werden, welches ein entsprechendes männliches oder weibliches Ende umfasst, das mit einer Schutzbrille (L) verbunden ist.

8. Kopfschutzhaube (1) nach einem der vorstehenden Ansprüche, **dadurch gekennzeichnet, dass** das mindestens eine Verbindungsmittel (5) in der Länge anpassbar ist.

9. Kopfschutzhaube (1) nach einem der vorstehenden Ansprüche, **dadurch gekennzeichnet, dass** sie ein System zum automatischen Anpassen der Größe der Haube umfasst, das an der Hinterseite der Haube platziert ist.

10. Set, umfassend:
- eine Kopfschutzhaube (1) nach einem der Ansprüche 1 bis 9, die integraler Bestandteil einer Schutzkleidung (8) für Reinräume ist, und
- eine Schutzbrille (L), die im verpackten Zustand und beim Anziehen von mindestens einem der ersten und zweiten Verbindungsmittel (5), die mit der Haube (1) verbunden sind, gehalten wird.

11. Verfahren zum Anziehen einer Schutzkleidung (8) für Reinräume, die mit einer Kopfschutzhaube nach einem der Ansprüche 1 bis 9 ausgestattet ist, wobei das Verfahren die Schritte umfasst:
- eines Überziehens, durch einen Benutzer, der Schutzkleidung (8) für Reinräume durch eine Öffnung dieser letzteren, mit Positionieren des Kopfes des Benutzers in der Haube (1), wobei eine Schutzbrille (L) von mindestens einem der ersten und zweiten Verbindungsmittel (5) an der Haube (1) gehalten wird, und
- eines Anpassens, durch den Benutzer, der Positionierung der Schutzbrille (L) vor der Gesichtsöffnung (4), die im Bereich seiner Augen vorgesehen ist.

12. Anziehverfahren nach Anspruch 11, das weiter einen Schritt des Verbindens der Schutzbrille (L) mit der Haube (1) über ein zweites Verbindungsmittel (5) umfasst, wenn die Schutzbrille (L) zuvor nur von einem ersten Verbindungsmittel (5) an der Haube (1) gehalten wird.

13. Verfahren nach Anspruch 11 oder 12, das weiter einen zusätzlichen Schritt des Schließens der Öffnung der Schutzkleidung (8) für Reinräume umfasst, zum Beispiel mittels einer Zuglasche oder jedem anderen passenden Verschlussmittel.

## Claims

1. Protective hood (1) for the head, being an integral part of a protective garment (8) for clean room, said hood (1) having:
- an external face (2) provided to be in contact with a surrounding environment,
- an internal face provided to be in contact with the head of a user,
- a head passage section (3),
- a facial opening (4) provided at the eye level of the user, and
- a first and a second connecting means (5) for a set of protective eyewear (L), said connecting means (5) being connected to said hood (1) respectively on both sides of said facial opening (4),
said hood **characterised in that**, in the packaged state and when worn, a set of protective eyewear (L) is attached to the hood (1) by at least one of said first and second connecting means.

2. Protective hood (1) for the head according to claim 1, **characterised in that** a said first connecting means and/or a said second connecting means (5) connected to said hood (1) is/are located on an axis which substantially horizontally traverses said facial opening (4).

3. Protective hood (1) for the head according to claim 1 or 2, **characterised in that** a said first connecting means and/or a said second connecting means is/are connected to said external face (2) or to said internal face of the hood (1).

4. Protective hood (1) for the head according to any one of the preceding claims, **characterised in that** a said first connecting means and/or a second connecting means (5) is/are connected to a rear part of said hood (1) or a side part of said hood (1).

5. Protective hood (1) for the head according to any one of the preceding claims, **characterised in that** a said first connecting means and/or a said second connecting means (5) has/have a first end (z) connected to said hood (1) and a second free end (a).

6. Protective hood (1) for the head according to any one of the preceding claims, **characterised in that** a said first connecting means and/or a said second connecting means (5) comprises/comprise an engagement member (6) arranged to be engaged within a corresponding engagement member (7) connected to a set of protective eyewear (L).

7. Protective hood (1) for the head according to claim 6, **characterised in that** said engagement member (6) comprises a male or female end arranged to be engaged within a corresponding engagement member (7) comprising a corresponding male or female end connected to a set of protective eyewear (L).

8. Protective hood (1) for the head according to any one of the preceding claims, **characterised in that** at least one connecting means (5) is adjustable in length.

9. Protective hood (1) for the head according to any one of the preceding claims, **characterised in that** it comprises a system for auto-adjustment of the size of the hood, located at the rear of the hood.

10. Assembly comprising:
- a protective hood (1) for the head being an integral part of a protective garment (8) for clean room according to any one of claims 1 to 9, and
- a set of protective eyewear (L) attached by at least one of said first and second connecting means (5) connected to said hood (1), in the packaged state and when worn.

11. Method for wearing a protective garment (8) for clean room, equipped with a protective hood for the head according to any one of claims 1 to 9, said method comprising the following steps:
- a user pulling on the protective garment (8) for clean room via an opening of the latter, positioning the head in said hood (1), a set of protective eyewear (L) being attached to the hood (1) by at least one of said first and second connecting means (5), and
- the user adjusting the position of the protective eyewear (L) in front of said facial opening (4) provided at eye level.

12. Wearing method according to claim 11, further comprising a step of connecting the protective eyewear (L) to the hood (1) by a second connecting means (5) when beforehand the protective eyewear (L) was only attached to the hood (1) by a first connecting means (5).

13. Method according to claim 11 or 12, further comprising an additional step of closing said opening of the protective garment (8) for clean room, for example by means of a pull-tab or any other adequate closing means.
